# FASCICULE DE BREVET EUROPEEN

(11) **EP 3 850 127 B1**
(45) Date de publication et mention de la délivrance du brevet: **01.06.2022**
(21) Numéro de dépôt: 19787028.0
(22) Date de dépôt: 12.09.2019
(51) Int. Cl.: C25B 3/00, C25B 3/26, C25B 9/19, C25B 11/02, C25B 11/043, C12P 7/04, C12P 7/40, C02F 1/461, C02F 3/00

(54) **RÉACTEUR BIO-ÉLECTROCHIMIQUE À DOUBLE BIO-ANODE, PROCÉDÉ DE RÉGÉNÉRATION ANODIQUE ET UTILISATION DU RÉACTEUR À L'ÉLECTROSYNTHÈSE MICROBIENNE**
BIOELEKTROCHEMISCHER REAKTOR MIT DOPPELTER BIOANODE, VERFAHREN ZUR ANODISCHEN REGENERATION UND VERWENDUNG DES REAKTORS FÜR DIE MIKROBIELLE ELEKTROSYNTHESE
BIOELECTROCHEMICAL REACTOR WITH DOUBLE BIOANODE, METHOD FOR ANODIC REGENERATION AND USE OF THE REACTOR FOR MICROBIAL ELECTROSYNTHESIS

(30) Priorité: 13.09.2018 FR 1858240
(43) Date de publication de la demande: 21.07.2021
(73) Titulaire: SUEZ Groupe, 92040 Paris la Défense Cedex (FR); Institut national de recherche pour l'agriculture, l'alimentation et l'environnement, 75007 Paris (FR); Centre national de la recherche scientifique, 75016 Paris (FR); Institut National Polytechnique de Toulouse, 31029 Toulouse (FR)
(72) Inventeur: BERGEL, Alain, 31500 TOULOUSE (FR); BERNET, Nicolas, 11590 CUXAC D'AUDE (FR); BLANCHET, Elise, 31570 BOURG ST BERNARD (FR); BOUCHEZ, Théodore, 91630 VILLEMOISSON (FR); ERABLE, Benjamin, 81500 GIROUSSENS (FR); ETCHEVERRY, Luc, 31450 MONTLAUR (FR); HUYARD, Alain, 78130 LES MUREAUX (FR); LE QUEMENER, Elie, 11100 NARBONNE (FR); MAURICRACE, Pierre, 78113 GRANDCHAMP (FR); MOREAU, Sylvain, 94370 SUCY EN BRIE (FR); TIAN, Jianghao, 91300 MASSY (FR); TRABLY, Eric, 11590 CUXAC D'AUDE (FR)
(74) Mandataire: Ipsilon
(86) Numéro de dépôt international: PCT/FR2019/052110
(87) Numéro de publication internationale: WO 2020/053529

(56) Documents cités:
- WO-A1-2017/222382
- CN-B- 103 922 487
- US-A1- 2014 069 806
- KORNEEL RABAEY ET AL: "Microbial electrosynthesis - revisiting the electrical route for microbial production", NATURE REVIEWS MICROBIOLOGY, vol. 8, no. 10, 1 octobre 2010 (2010-10-01), pages 706-716, XP055205190, ISSN: 1740-1526, DOI: 10.1038/nrmicro2422
- Anonymous: "Auxiliary electrode - Wikipedia", , 8 septembre 2018 (2018-09-08), XP055588509, Extrait de l'Internet: URL:https://web.archive.org/web/2018090805 2800/https://en.wikipedia.org/wiki/Auxilia ry_electrode [extrait le 2019-05-14]

## Description

### DOMAINE DE L'INVENTION

La présente invention concerne le domaine bio-électrochimique, et concerne plus particulièrement les systèmes et les procédés de synthèse électrochimique mettant en œuvre des réacteurs bio-électrochimiques c'est-à-dire des dispositifs électrochimiques dont l'une au moins des électrodes appelée bio-électrode, est au contact de microorganismes.

### ETAT DE LA TECHNIQUE

Ces dispositifs de synthèses bio-électrochimiques permettent notamment, à partir de déchets organiques, de produire des molécules organiques telles que des acides organiques et/ou des alcools.

En particulier, il a été mis au point récemment un tel dispositif bio-électrochimique, qui comporte à la fois une bio-anode et une bio-cathode, l'électrolyte du compartiment anodique ainsi que l'électrolyte du compartiment cathodique renfermant des microorganismes en suspension ou sous forme de biofilm(s) (WO2016/051064). Dans ce dispositif, l'activité de la bio-cathode est optimisée en vue de la production d'espèces chimiques particulières dans l'électrolyte, tels que les acides acétique, lactique et/ou propionique ou des alcools. Ces synthèses de molécules organiques par voie microbienne, impliquant en particulier des réactions d'oxydo-réducttion électrochimiques, sont réalisées grâce à des bactéries électro-actives présentes à la surface de l'électrode.

Un des problèmes actuels à résoudre est d'améliorer la fiabilité et la durabilité de ces dispositifs bio-électrochimiques, en vue d'applications au stade industriel.

Plus particulièrement dans le dispositif mentionné, un des objectifs est d'augmenter la durabilité de la bio-anode, c'est-à-dire de maintenir ses performances (caractérisées par des rendements acceptables notamment dans un contexte industriel) sur des durées plus longues. Il a en effet été constaté que l'activité de cette bio-anode diminue considérablement après quelques semaines de fonctionnement. Ce phénomène a été défini comme le « vieillissement » de la bio-anode, probablement dû à un colmatage du biofilm sur cette électrode. En effet, un biofilm composé de bactéries électro-actives (notamment du genre *Geobacter*) est nécessaire pour le fonctionnement de la bio-anode. D'autres microorganismes non électro-actifs se développent également sur ce biofilm et inhibent ainsi son activité électro-catalytique. Le dépôt de particules non-solubles aggrave encore cet effet.

Par ailleurs il existe un besoin pour des dispositifs et procédés qui puissent être déployés à l'échelle industrielle, c'est-à-dire qu'ils puissent traiter des volumes d'effluents tels que ceux traités aujourd'hui à l'échelle industrielle. Des problèmes spécifiques se posent lors de l'augmentation de la taille des réacteurs pour conserver les niveaux d'activité observés en laboratoire : notamment le volume du réacteur augmente en général de manière plus conséquente que la surface active des électrodes. En outre, étant donné les plages de fonctionnement des cellules électrochimiques microbiennes, il est important que la cellule permette de contrôler le potentiel de l'anode avec suffisamment de précision.

Ce problème est notamment évoqué par Logan et al (Environ. Sci. Technol. Lett. 2015, 2, 206-214), qui recommandent de conserver un rapport volume du réacteur/surface de la cathode pour pallier ce problème. Cependant, Logan *et al.* ne proposent pas de matériaux ni de configuration particulière pour la cathode.

Un autre objectif est d'améliorer la stabilité de la bio-cathode.

Cheng et al. (Environ. Sci. Technol. 2006, 40, 5426-2432) proposent d'optimiser la génération du courant en faisant varier la distance entre les électrodes. Dans leur système les deux électrodes en tissus de carbone.

Cependant, Cheng *et al.* proposent un système MFC (Microbial Fuel Cell) à une seule chambre qui enferme une cathode Pt/C abiotique. Leur étude se focalise sur la génération d'électricité par l'oxydation de glucose à l'anode.

L'art antérieur décrit ci-dessus ne propose pas de solutions aux problèmes spécifiques des réacteurs bio-électrochimiques comportant à la fois une bio-anode et une bio-cathode. US 2014/069806 A1, RABAEY et al, Nature Reviews Microbiology, 2010, 8(10), 706 et CN 103 922 487 B divulguent des réacteurs bio-électrochimiques. WO 2017/222382 A1 divulgue un réacteur électrochimique comprenant un compartiment anodique, un compartiment cathodique et un compartiment inter-membranaire.

### BUTS DE L'INVENTION

Un premier but de l'invention est donc de pallier les inconvénients de l'art antérieur en proposant un réacteur bio-électrochimique, notamment dans un dispositif de synthèse bio-électrochimique, un système permettant de garantir son fonctionnement le plus stable possible, et sur de longues périodes.

Un autre but de l'invention est de proposer un réacteur bio-électrochimique dont la structure permet la régénération ou la restauration de l'activité électrochimique d'une bio-électrode « vieillissante », sans arrêter le fonctionnement du dispositif de synthèse.

### DESCRIPTION DE L'INVENTION

A cet effet, la présente invention concerne un réacteur bio-électrochimique comprenant
- un compartiment anodique comportant au moins deux anodes, dénommées bio-anodes, et un électrolyte anodique comprenant des microorganismes électro-actifs anodiques,
- un compartiment cathodique comportant au moins une cathode, dénommée bio-cathode, et un électrolyte cathodique comprenant des microorganismes électro-actifs cathodiques,
- le compartiment anodique étant séparé du compartiment cathodique par, en allant du compartiment anodique vers le compartiment cathodique, une membrane échangeuse de cations et une membrane échangeuse d'anions, lesdites membranes échangeuses de cations et d'anions étant séparées l'une de l'autre par un compartiment intermembranaire,
des moyens d'application d'une différence de potentiel entre les bio-anodes connectées entre-elles et la ou les bio-cathode(s),
les bio-anodes et bio-cathode(s) présentant des surfaces actives telles que la surface active totale de la ou des bio-cathode(s) est supérieure à la surface active totale des au moins deux bio-anodes.

Au sens de l'invention, une « bio-électrode » (« bio-anode » ou « bio-cathode ») est une électrode recouverte au moins en partie d'un biofilm bactérien comprenant des organismes électro-actifs, c'est-à-dire recouverte au moins sur une partie de sa surface immergée dans l'électrolyte par un biofilm bactérien. Selon un mode de réalisation, la totalité de la surface immergée de la bio-électrode est recouverte de biofilm. Alternativement, selon un autre mode de réalisation, une partie seulement de la surface de la bio-électrode est recouverte de biofilm. Dans ce dernier mode de réalisation, la surface recouverte de biofilm est suffisante pour générer l'activité recherchée, notamment dans le cas d'une oxydation d'hydrolysats de déchets organiques ou d'une synthèse bio-électrochimique.

Selon un mode de réalisation avantageux de l'invention, le réacteur bio-électrochimique comporte deux bio-anodes et une bio-cathode. Cependant, l'invention peut concerner tout réacteur bio-électrochimique comportant plus de deux bio-anodes et plusieurs bio-cathodes.

La présence de deux bio-anodes dans le compartiment anodique permet notamment leur utilisation en alternance : en particulier lorsque l'une est « vieillissante », c'est-à-dire lorsque son activité électrochimique décroit, cela permet de la remplacer ou de la régénérer. En fonctionnement normal les deux bio-anodes sont connectées électriquement, généralement en parallèle. Selon un mode de réalisation ces deux bio-anodes sont sensiblement au même potentiel, en particulier lorsque celles-ci présentent une géométrie identique.

Le compartiment inter-membranaire est apte à recueillir les ions ou molécules produites dans les compartiments anodique et/ou cathodique.

Par surface active d'une bio-électrode (ici bio-anode ou bio-cathode) on entend la surface exposée à l'électrolyte, cette surface étant polarisée. Selon l'invention, la bio-cathode présente une plus grande inertie du fait d'une surface active supérieure à la surface active totale des deux bio-anodes, ce qui permet de garantir un potentiel de cathode particulièrement stable. En effet, en fonctionnement, une fois que la cathode a atteint son potentiel de travail, la grande stabilité du potentiel de la cathode permet en pratique de mieux contrôler le potentiel anodique en faisant varier la différence de potentiel entre la bio-cathode et les bio-anodes, et sans avoir à recourir à une électrode de référence. Un tel système permet ainsi un contrôle fin du potentiel anodique et donc l'optimisation de l'activité du biofilm anodique.

De manière avantageuse, les bio-anodes sont amovibles, et sont ainsi aptes à être régénérées séparément et /ou remplacées. En variante, les bio-anodes ne sont pas nécessairement amovibles et peuvent être régénérées selon les procédés décrits dans les demandes parallèles déposées le même jour que la présente demande de brevet, sous priorité des demandes françaises FR 18 58236 et FR 18 58238non encore publiées.

Selon un mode de réalisation particulier, le réacteur bio-électrochimique est un réacteur d'électrosynthèse microbienne. Dans ce mode de réalisation, le réacteur est caractérisé en ce que le compartiment anodique comporte un ou plusieurs ports d'injection de substrat carboné organique, tels que des hydrolysats de biodéchets organiques, le compartiment cathodique comporte un ou plusieurs ports d'injection de CO₂ ou d'introduction d'une source carbonée organique ou minérale et le compartiment inter-membranaire un dispositif de soutirage des molécules synthétisées au sein dudit réacteur.

En ce qui concerne les bio-électrodes :
- la bio-cathode est, de préférence, une électrode tri-dimensionnelle, notamment comprenant un matériau granulaire ou se présentant sous la forme générale d'un treillis. La bio-cathode peut, par exemple, comprendre des grains de carbone disposés dans un contenant en acier inox.
- les bio-anodes se présentent, de préférence, sous la forme générale d'un panneau, en particulier plan ou arrondi. Les bio-anodes sont, par exemple, formées d'un tissu ou feutre de carbone, maintenu dans un cadre métallique, de préférence un cadre en acier inox.

Par électrode tri-dimensionnelle, on entend ici, une électrode dont les dimensions géométriques épaisseur/hauteur/largeur sont telles que son épaisseur corresponde à sa plus petite dimension et soit supérieure ou égale à 1/10 de chacune de ses deux autres dimensions. Par opposition, on entend par forme générale d'un "panneau", une électrode présentant une épaisseur inférieure à 1/10 de chacune de ses deux autres dimensions, hauteur et largeur.

Les microorganismes électro-actifs sont des microorganismes capables d'interagir directement avec une électrode, ce sont ici typiquement des microorganismes anaérobies. Les microorganismes diffèrent en fonction de l'électrode sur laquelle ils se développent sous forme de biofilm, et des caractéristiques de l'électrolyte dans lequel ils sont immergés. Par exemple, lorsque des eaux usées ou des hydrolysats de bio-déchets sont injectés dans l'électrolyte anodique, on observe une population abondante affiliée au genre *Geobacter.* Par contre, dans un milieu salin, d'autres genres tels que *Geoalkalibacter* ou *Desulforomonas* peuvent devenir dominants. Ainsi, lorsque les micro-organismes sont situés sur l'anode, on parle de microorganismes électro-actifs anodiques, tandis que lorsque les micro-organismes sont situés sur la cathode, on parle de microorganismes électro-actifs cathodiques ou électrotrophes.

Le réacteur, selon l'invention, peut en outre comprendre des moyens de régulation du pH, de la température, et/ou du niveau d'électrolyte, de préférence, dans chacun des compartiments anodique et cathodique.

La présente invention concerne également un procédé de régénération de l'activité des bio-anodes du réacteur, tel que décrit ci-dessus, comprenant :
une étape de retrait d'au moins une des bio-anodes du compartiment anodique, étant entendu qu'au moins une bio-anode est laissée dans le compartiment anodique, et
une étape d'introduction dans le compartiment anodique d'au moins une anode non colonisée par des microorganismes électro-actifs, le réacteur étant maintenu en fonctionnement par application d'une différence de potentiel entre la bio-cathode et la bio-anode restant dans le compartiment anodique.

Selon un premier mode de réalisation, l'anode non colonisée est l'anode retirée du compartiment, ayant subi un nettoyage. Dans ce mode de réalisation, le procédé comprend donc :
une étape de retrait d'au moins une des bio-anodes du compartiment anodique, étant entendu qu'au moins une bio-anode est laissée dans le compartiment anodique,
une étape de nettoyage (mécanique, chimique ou thermique), à l'extérieur du réacteur, de ladite bio-anode retirée, puis
sa réintroduction dans le compartiment anodique, le réacteur étant maintenu en fonctionnement par application d'une différence de potentiel entre la bio-cathode et la bio-anode restant dans le compartiment anodique.

Selon un autre mode de réalisation, l'anode non colonisée par des microorganismes électro-actifs est une anode neuve. Selon ce mode de réalisation, le procédé de régénération de l'activité des bio-anodes du réacteur, tel que décrit ci-dessus, comprend le remplacement d'une des bio-anodes du compartiment anodique, par une anode non colonisée par des microorganismes électro-actifs, telle qu'une anode « neuve », le réacteur étant maintenu en fonctionnement par application d'une différence de potentiel entre la bio-cathode et la bio-anode restant dans le compartiment anodique.

Le réacteur selon l'invention permet ainsi la régénération ou la restauration de l'activité électrochimique anodique « vieillissante », sans arrêter le fonctionnement du dit réacteur.

Le réacteur selon la présente invention trouve une utilisation intéressante pour l'électrosynthèse d'acides organiques et/ou d'alcools à partir de déchets organiques. Les déchets organiques utilisés dans l'invention sont typiquement choisis parmi : des hydrolysats de bio-déchets, des boues hydrolysées de stations d'épuration, différentes fractions liquides organiques de stations d'épuration, des eaux usées urbaines après décantation primaire, des effluents industriels organiques, des effluents d'industries agro-alimentaires, des digestats de stations d'épuration, ou un mélange de plusieurs des substrats ci-dessus

L'électrolyte du compartiment anodique renferme ainsi de tels substrats carbonés organiques sous forme liquide, introduits soit bruts, soit dilués dans un électrolyte de base synthétique. Dans ce compartiment anodique la teneur en matières organiques quantifiées par la mesure de la DCO (demande chimique en oxygène) est avantageusement comprise entre 0,01 et 200 g/L, de préférence entre 0,1 et 20 g/L, de préférence encore entre 0,1 et 5 g/L.

La bio-cathode est avantageusement conditionnée par introduction d'un inoculum dans l'électrolyte cathodique.

Dans un mode de réalisation préféré, l'inoculum est préparé à partir d'une boue de digesteur anaérobie, ayant éventuellement subi un prétraitement visant à inactiver les microorganismes méthanogènes. Ainsi, cette boue de digesteur peut subir un traitement thermique à une température et pendant une durée suffisante pour inactiver les microorganismes méthanogènes.

Le prétraitement peut également comprendre l'enrichissement du déchet en microorganismes d'intérêt. Cette étape peut notamment comprendre l'ajout d'hydrogène et de dioxyde de carbone, par exemple dans une fiole fermée en mode discontinu. Au sens de l'invention, les microorganismes d'intérêt sont les microorganismes responsables de la bio-électrosynthèse, et comprennent par exemple des bactéries capables d'utiliser les électrons ou l'hydrogène générés à la cathode pour synthétiser les composés désirés (tels que des acides organiques ou alcools).

La culture résultant de cet enrichissement peut être utilisée directement et introduite dans le compartiment cathodique au démarrage du réacteur.

L'électrolyte du compartiment cathodique renferme un électrolyte et une source de carbone, injecté sous forme de gaz : tel que du CO₂, du biogaz, ou du syngas, et/ou introduit en solution sous la forme de carbone organique : par exemple acétate et/ou sous la forme de carbone minéral : par exemple un bicarbonate.

### BREVE DESCRIPTION DES FIGURES

D'autres caractéristiques et avantages de l'invention apparaîtront dans la description ci-dessous d'exemples de réalisation, non limitatifs, en référence aux schémas annexés, dans lesquels :
La figure 1 schématise un réacteur bio-électrochimique, selon l'invention, montrant les différents compartiments et la localisation des bio-électrodes ;
La figure 2 est un schéma montrant les éventuels systèmes de régulation présents dans un réacteur selon l'invention (les électrodes n'étant pas représentées pour plus de clarté) ;
La figure 3 est une vue de face de la bio-cathode, la figure 3A étant un schéma de profil de la bio-cathode de la figure 3 ;
La figure 4 est une vue de face d'une bio-anode, la figure 4A étant un schéma de profil de ladite bio-anode de la figure 4 ;
La figure 5 est une vue de dessus en perspective d'un réacteur selon l'invention ;
La figure 6 montre l'intérieur des compartiments du réacteur de la figure 5 ;
La figure 7 présente un diagramme montrant la densité de courant anodique du réacteur de la figure 5, en fonction du temps, avant et après régénération d'une des bio-anodes du compartiment anodique.

### EXEMPLES

En se référant aux figures, le réacteur selon l'invention se compose de manière générale de trois compartiments séparés par des membranes échanges d'ions, à savoir : un compartiment anodique 11 qui renferme deux bio-anodes 12 et 13 reliées électriquement à l'extérieur du réacteur, et un compartiment cathodique 21 comprenant la bio-cathode 22, un compartiment anodique 11 séparé du compartiment cathodique 21 par un compartiment intermembranaire 30.

Une membrane échangeuse de cations 31 sépare le compartiment anodique 11 du compartiment intermembranaire 30 et une membrane échangeuse d'anions 32 sépare le compartiment cathodique 21 du compartiment intermembranaire 30.

Le compartiment anodique 11 renferme un électrolyte anodique 14 comprenant des microorganismes électro-actifs anodiques. Le compartiment cathodique 21 renferme un électrolyte cathodique 24 comprenant des microorganismes électro-actifs cathodiques.

Une différence de potentiel 2 est appliquée entre la bio-cathode 22 et les deux bio-anodes 12 et 13. Le compartiment anodique comporte notamment un port 3 d'injection de substrat carboné organique.

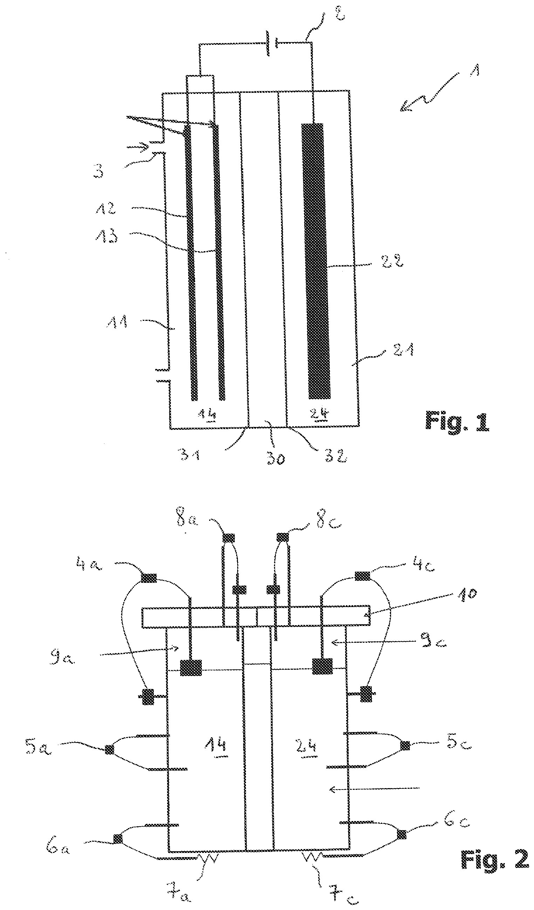

Divers systèmes de régulation, dans ledit réacteur selon l'invention, peuvent être incorporés audit réacteur et sont schématisés sur la figure 2. On peut avoir, notamment, un système de régulation du niveau du liquide anodique 4a et/ou cathodique 4c, un système de régulation du pH anodique 5a et/ou cathodique 5c, un système de régulation de température du compartiment anodique 6a et/ou du compartiment cathodique 6c au moyen, par exemple, d'une résistance chauffante 7a et/ou 7c. Enfin, un système de régulation de pression de la phase gazeuse 9a ou 9c peut être prévu dans chacun des compartiments d'électrodes soit 8a anodique ou 8c cathodique. En effet, le réacteur est fermé par un couvercle 10.

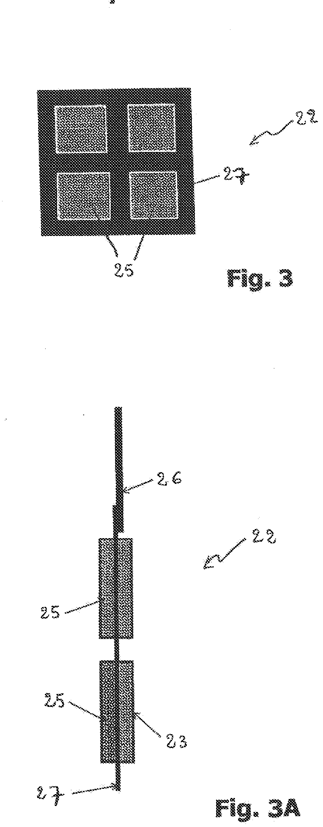

Un exemple de structure de la cathode est présenté aux figures 3 et 3A.

La bio-cathode 22 se compose d'un cadre 27 de dimension 30x30 cm délimitant quatre logements sur l'exemple présenté ici. Ces logements incorporent des paniers métalliques 23 d'épaisseur comprise entre 4 et 5 cm dans lesquels sont disposées des granules de carbone 25. Le cadre métallique 27 est connecté à un collecteur de courant 26 surmontant ledit cadre.

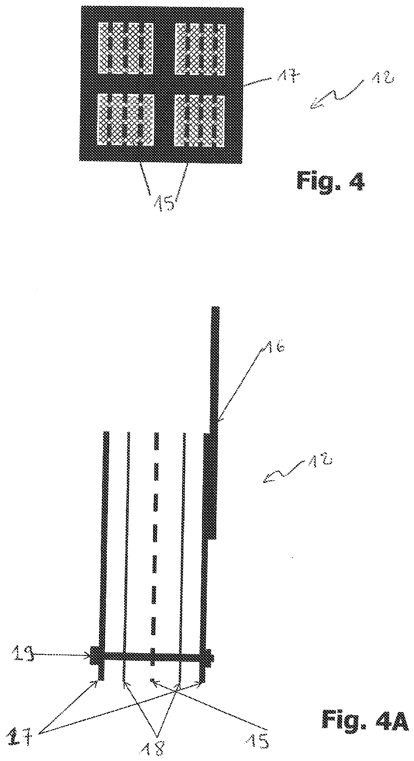

Un exemple de structure d'une bio-anode est présenté aux figures 4 et 4A (vue éclatée).

Par exemple, la bio-anode 12 se compose d'un cadre métallique 17 se décomposant en deux parois parallèles qui enserrent entre elles deux grilles inox parallèles 18 logeant entre elles un tissu de carbone 15. Ce tissu de carbone 15 peut se présenter sous la forme d'un élément unique ou sous la forme de bandes de tissu disposées parallèlement comme schématisé sur la figure 4. L'ensemble est maintenu, par exemple, au moyen de vis 19.

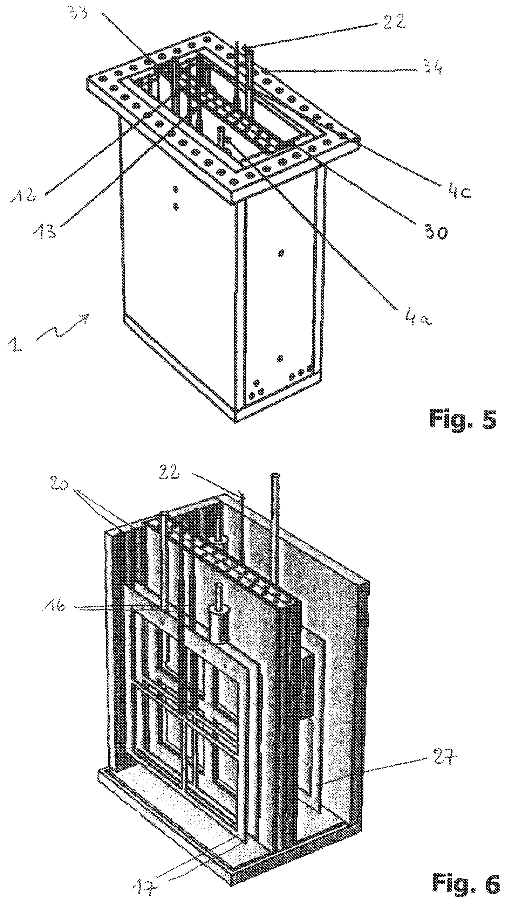

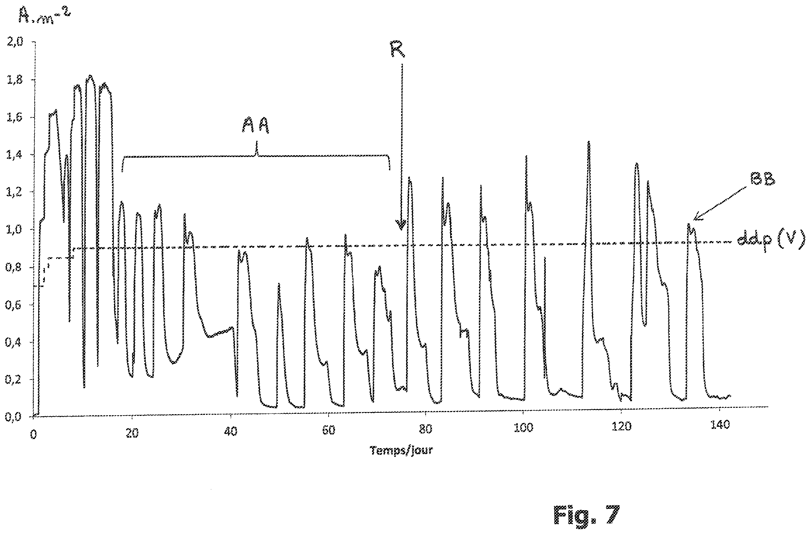

Un descriptif plus précis du réacteur 1 bio-électrochimique selon l'invention est schématisé aux figures 6 et 7.

### Exemple 1

Le réacteur 1 bio-électrochimique selon l'invention schématisé aux figures 5 et 6, a été conçu pour mimer les conditions industrielles. Ce réacteur comprend trois compartiments séparés par deux membranes échangeuses d'ions : un compartiment anodique 11 qui enferme deux bio-anodes 12 et 13 (reliées électriquement à l'extérieur du réacteur). Ce compartiment est séparé par une membrane 31 échangeuse de cations, d'un compartiment intermembranaire 30 qui est lui-même séparé par une membrane 32 échangeuse d'anions du compartiment cathodique 21 qui enferme la bio-cathode 22. Les volumes de ces trois compartiments sont de 5,25 L, 2 L et 5,25 L respectivement.

La taille de chaque bio-anode 12, 13 est de 30X30 cm et d'épaisseur inférieure à 1 cm. Les surfaces actives de ces deux bio-anodes est ainsi de 0,36 m², si l'on considère les quatre faces des deux bio-anodes. La bio-cathode 22 comprend un volume de 1,2 L de grains de carbone, qui présentent une surface active d'environ 3 m2, c'est-à-dire de l'ordre de 10 fois la surface totale active des bio-anodes.

Ces bio-électrodes sont connectées à un potentiostat (BioLogic^{®}, France, VMP3 non représenté, piloté par le logiciel EC-Lab), une différence de potentiel de 1,1 V étant imposée entre les bio-anodes et la bio-cathode.

Des électrodes de référence 33, 34 peuvent être présentes respectivement dans les compartiments anodique 11 et/ou cathodique 12. Dans un réacteur à échelle industrielle, ces électrodes de référence peuvent être absentes.

L'électrolyte cathodique 24 est le milieu BMP modifié avec 30 g/L de NaHCO₃. L'électrolyte anodique 14 de base est composé de 12,5 g/L de Na₂HPO₄.7H₂O, 3 g/L de KH₂PO₄, 0,5 g/L de NaCI, 1 g/L de NH₄Cl et 30 g/L de NaHCO₃. L'électrolyte du compartiment intermembranaire 30 est composé de 35 g/L de KCI et 32,6 g/L de KH₂PO₄.

Le pH de l'électrolyte anodique est maintenu à 7 par injection automatique d'une solution de K₂CO₃ dans le compartiment anodique. Les biodéchets utilisés sont des hydrolysats, très chargés en matières organiques, par exemple dont la valeur de DCO est comprise entre 100 et 150 g/L. Ces hydrolysats sont introduits dans l'électrolyte anodique par injection d'un volume de 10 à 20 mL, soit quotidiennement, soit lorsque le courant anodique chute au-dessous de 0,5 A/m2 environ.

Un dispositif de collecte (non représenté) des molécules synthétisées peut être relié au compartiment intermembranaire.

Une légère surpression (par exemple 20-30 mbar) peut être maintenue dans le ciel gazeux des compartiments anodique et cathodique, permettant d'éviter une entrée d'air dans ces compartiments.

### Préparation d'inoculum pour la bio-cathode

Dans le cas d'une application du procédé de l'invention à l'électrosynthèse d'acides organiques ou d'alcools, l'inoculum pour la bio-cathode 22 peut être préparé à partir d'une boue de digesteur anaérobie. La préparation consiste à appliquer des traitements pour d'une part inactiver les microorganismes méthanogènes, concurrents de la réaction souhaitée, et d'autre part enrichir la boue en microorganismes d'intérêt.

La première étape consiste à traiter thermiquement l'inoculum (à 90°C pendant 20 minutes) ce qui a pour conséquence d'inactiver les méthanogènes.

La seconde étape consiste à enrichir la boue en microorganismes d'intérêt par un apport d'hydrogène et de dioxyde de carbone dans une fiole fermée en mode discontinu. Cette opération peut être renouvelée deux fois. Les microorganismes d'intérêt comprennent ici des bactéries capables d'utiliser les électrons ou l'hydrogène générés à la cathode pour synthétiser les composés désirés (acides organiques ou alcools).

La culture résultant de cet enrichissement peut être utilisée directement et introduite dans le compartiment cathodique 21 au démarrage du réacteur.

### Exemple 2 - Régénération d'une bio-anode

Le réacteur, tel que décrit dans l'exemple 1, a été mis en fonctionnement pendant une durée de 140 jours. Une différence de potentiel de 0,9 V a été appliquée entre, d'une part les bio-anodes connectées électriquement entre-elles (disposées en parallèle), et d'autre part la bio-cathode.

Afin de quantifier l'activité d'une bio-anode, la méthode la plus utilisée est de mesurer la densité de courant maximum qu'elle est capable de produire en présence d'un substrat organique. La densité de courant aux bio-anodes a ainsi été suivie en fonction du temps (voir la courbe de la figure 7 présentant la densité de courant en traits pleins).

Après 18 jours environ, on constate une diminution de cette densité de courant, signe d'un vieillissement des bio-anodes (plage A-A sur la figure 7). Il a alors été procédé à la régénération (flèche R) d'une de ses bio-anodes selon le procédé suivant :
Le cadre 17 et le collecteur de courant 16 d'une des bio-anodes amovibles ont été retirés du compartiment anodique 11 par coulissement dans une des gorges 20 (voir figure 6) nettoyé avec un détergent puis séché, la grille 18 en inox et les tissus de carbone 15 ont été remplacés par des matériaux neufs.

La nouvelle bio-anode renouvelée a ensuite été replacée dans la position la plus proche de la membrane 31, l'autre bio-anode ayant été décalée dans l'autre gorge, plus proche de la paroi extérieure du réacteur.

On voit nettement qu'après ce remplacement d'une des bio-anodes, l'activité reprend pendant au moins une quarantaine de jours. On note à nouveau un vieillissement à partir du pic B-B sur la figure 7. On peut alors remplacer la seconde bio-anode comme présenté ci-dessus pour la première bio-anode.

## Revendications

1. Réacteur (1) bio-électrochimique comprenant
- un compartiment anodique (11) comportant au moins deux anodes (12, 13), dénommées bio-anodes, et un électrolyte anodique (14) comprenant des microorganismes électro-actifs anodiques,
- un compartiment cathodique (21) comportant au moins une cathode, dénommée bio-cathode (22), et un électrolyte cathodique (24) comprenant des microorganismes électro-actifs cathodiques,
- le compartiment anodique (11) étant séparé du compartiment cathodique (21) par, en allant du compartiment anodique vers le compartiment cathodique, une membrane échangeuse de cations (31) et une membrane échangeuse d'anions (32), lesdites membranes échangeuses de cations et d'anions étant séparées l'une de l'autre par un compartiment inter-membranaire (30),
des moyens d'application d'une différence de potentiel entre les bio-anodes connectées entre-elles et la ou les bio-cathode(s),
les bio-anodes et bio-cathode(s) présentant des surfaces actives telles que la surface active totale de la ou des bio-cathode(s) (22) est supérieure à la surface active totale des deux bio-anodes (12, 13).

2. Réacteur selon la revendication 1, **caractérisée en ce que** les bio-anodes (12, 13) sont amovibles.

3. Réacteur selon la revendication 1 ou 2, **caractérisé en ce que** ledit réacteur est un réacteur d'électrosynthèse microbienne, le compartiment anodique (11) comportant un ou plusieurs ports (3) d'injection de substrat carboné organique, tels que des hydrolysats de bio-déchets organiques, le compartiment cathodique (21) comportant un ou plusieurs ports d'injection de CO₂ ou d'introduction d'une source carbonée organique ou minérale et le compartiment inter-membranaire (30) un dispositif de soutirage des molécules synthétisées au sein dudit réacteur.

4. Réacteur selon l'une quelconque des revendications précédentes, **caractérisée en ce que** la bio-cathode (22) est une électrode dont les dimensions géométriques épaisseur/hauteur/largeur sont telles que son épaisseur corresponde à sa plus petite dimension et soit supérieure ou égale à 1/10 de chacune de ses deux autres dimensions.

5. Réacteur selon la revendication 4, **caractérisé en ce que** la bio-cathode (22) comprend un matériau granulaire ou se présente sous la forme générale d'un treillis.

6. Réacteur selon la revendication 4, **caractérisé en ce que** la bio-cathode comprend des grains de carbone disposés dans un contenant en acier inox.

7. Réacteur selon l'une quelconque des revendications précédentes, **caractérisé en ce que** les bio-anodes (12, 13) se présentent sous la forme générale d'un panneau, notamment plan ou arrondi, ou on entend par forme générale d'un panneau, une électrode présentant une épaisseur inférieure à 1/10 de chacune de ses deux autres dimensions, hauteur et largeur.

8. Réacteur selon la revendication 7, **caractérisé en ce que** les bio-anodes (12, 13) sont formées d'un tissu ou feutre de carbone, maintenu dans un cadre métallique, de préférence un cadre en acier inox.

9. Réacteur selon l'une quelconque des revendications précédentes, **caractérisé en ce qu'**il comporte des moyens de régulation du pH, de la température, et/ou du niveau d'électrolyte.

10. Procédé de régénération de l'activité des bio-anodes du réacteur selon l'une quelconque des revendications 2 à 9, comprenant :
une étape de retrait d'au moins une des bio-anodes (12, 13) du compartiment anodique (11), étant entendu qu'au moins une bio-anode est laissée dans le compartiment anodique,
une étape de nettoyage, à l'extérieur du réacteur (1), de ladite ou desdites bio-anodes retirées, puis
leur réintroduction dans le compartiment anodique (11),
le réacteur étant maintenu en fonctionnement par application d'une différence de potentiel entre la bio-cathode et la bio-anode restant dans le compartiment anodique.

11. Procédé de régénération de l'activité des bio-anodes du réacteur selon l'une quelconque des revendications 2 à 9, comprenant :
le remplacement d'au moins une des bio-anodes (12, 13) du compartiment anodique (11), par une anode non colonisée par des microorganismes électro-actifs, étant entendu qu'au moins une bio-anode est laissée dans le compartiment anodique,
le réacteur étant maintenu en fonctionnement par application d'une différence de potentiel entre la bio-cathode et la bio-anode restant dans le compartiment anodique.

12. Utilisation du réacteur selon l'une quelconque des revendications 1 à 9 pour l'électrosynthèse d'acides organiques et/ou d'alcools à partir de déchets organiques.

13. Utilisation selon la revendication 12, **caractérisée en ce que** les déchets organiques sont choisis parmi : des hydrolysats de bio-déchets, des boues hydrolysées de stations d'épuration, différentes fractions liquides organiques de stations d'épuration, des eaux usées urbaines après décantation primaire, des effluents industriels organiques, des effluents d'industries agro-alimentaires, des digestats de stations d'épuration, ou un mélange de plusieurs de ceux-ci.

## Patentansprüche

1. Bioelektrochemischer Reaktor (1), umfassend:
- eine Anodenkammer (11), umfassend mindestens zwei Anoden (12, 13), bezeichnet als Bioanoden, und einen Anodenelektrolyt (14), umfassend anodische elektroaktive Mikroorganismen,
- eine Kathodenkammer (21), umfassend mindestens eine Kathode, bezeichnet als Biokathode (22), und einen Kathodenelektrolyt (24), umfassend kathodische elektroaktive Mikroorganismen,
- wobei die Anodenkammer (11) von der Kathodenkammer (21), reichend von der Anodenkammer zur Kathodenkammer, durch eine Kationenaustauschmembran (31) und eine Anionenaustauschmembran (32) getrennt ist, wobei die Kationen- und Anionenaustauschmembrane voneinander durch eine Zwischenmembrankammer (30), Mittel zur Anwendung einer Spannungsdifferenz zwischen den miteinander verbundenen Bioanoden und der oder den Biokathode(n) getrennt sind,
wobei die Bioanoden und Biokathode(n) aktive Flächen aufweisen, so dass die gesamte aktive Fläche der Biokathode(n) (22) grösser als die gesamte aktive Fläche der zwei Bioanoden (12, 13) ist.

2. Reaktor nach Anspruch 1, **dadurch gekennzeichnet, dass** die Bioanoden (12, 13) entfernbar sind.

3. Reaktor nach Anspruch 1 oder 2, **dadurch gekennzeichnet, dass** der Reaktor ein Reaktor für die mikrobielle Elektrosynthese ist, wobei die Anodenkammer (11) einen oder mehrere Anschlüsse (3) zur Injektion von organischem kohlenstoffhaltigem Substrat, wie z. B. Hydrolysaten von organischen Bioabfällen, umfasst, wobei die Kathodenkammer (21) einen oder mehrere Anschlüsse zur Injektion von CO₂ oder zur Einführung einer organischen oder mineralischen kohlenstoffhaltigen Quelle und die Zwischenmembrankammer (30) eine Vorrichtung zum Abstich der synthetisierten Moleküle innerhalb des Reaktors umfasst.

4. Reaktor nach einem der vorhergehenden Ansprüche, **dadurch gekennzeichnet, dass** die Biokathode (22) eine Elektrode ist, deren geometrische Abmessungen Dicke/Höhe/Breite derart sind, dass ihre Dicke ihren kleinsten Abmessung entspricht und grösser oder gleich 1/10 jeder ihrer zwei anderen Abmessungen ist.

5. Reaktor nach Anspruch 4, **dadurch gekennzeichnet, dass** die Biokathode (22) ein körniges Material umfasst oder die allgemeine Form eines Gitters aufweist.

6. Reaktor nach Anspruch 4, **dadurch gekennzeichnet, dass** die Biokathode Kohlenstoffkörner aufweist, die in einem Behälter aus Edelstahl angeordnet sind.

7. Reaktor nach einem der vorhergehenden Ansprüche, **dadurch gekennzeichnet, dass** die Bioanoden (12, 13) die allgemeine Form einer Platte aufweisen, insbesondere eben oder abgerundet, wobei unter der allgemeinen Form einer Platte eine Elektrode verstanden wird, die eine Dicke von weniger als 1/10 jeder ihrer zwei anderen Abmessungen Höhe und Breite aufweist.

8. Reaktor nach Anspruch 7, **dadurch gekennzeichnet, dass** die Bioanoden (12, 13) aus einem Kohlenstoffgewebe oder -filz hergestellt sind, gehalten in einem Metallrahmen, vorzugsweise einem Rahmen aus Edelstahl.

9. Reaktor nach einem der vorhergehenden Ansprüche, **dadurch gekennzeichnet, dass** er Mittel zur Regulierung des pH, der Temperatur und/oder des Elektrolytniveaus umfasst.

10. Verfahren zur Regenerierung der Aktivität der Bioanoden des Reaktors nach einem der Ansprüche 2 bis 9, umfassend:
einen Schritt des Rückziehens mindestens einer der Bioanoden (12, 13) aus der Anodenkammer (11), wobei davon ausgegangen wird, dass mindestens eine Bioanode in der Anodenkammer gelassen wird,
einen Schritt des Reinigens, Außerhalb des Reaktors (1), der zurückgezogenen Bioanode(n), dann
ihr Wiedereinführen in die Anodenkammer (11),
wobei der Reaktor durch das Anwenden einer Potenzialdifferenz zwischen der Biokathode und der Bioanode, die in der Anodekammer verbleibt, in Betrieb gehalten wird.

11. Verfahren zum Regenerieren der Aktivität der Bioanoden des Reaktors nach einem der Ansprüche 2 bis 9, umfassend:
Ersetzen mindestens einer der Bioanoden (12, 13) der Anodenkammer (11) durch eine Anode, die nicht von elektroaktiven Mikroorganismen kolonisiert ist, wobei davon ausgegangen wird, dass mindestens eine Bioanode in der Anodenkammer gelassen wird,
wobei der Reaktor durch das Anwenden einer Potenzialdifferenz zwischen der Biokathode und der Bioanode, die in der Anodekammer verbleibt, in Betrieb gehalten wird.

12. Verwendung des Reaktors nach einem der Ansprüche 1 bis 9 zur Elektrolyse von organischen Säuren und/oder Alkoholen auf der Grundlage von organischen Abfällen.

13. Verwendung nach Anspruch 12, **dadurch gekennzeichnet, dass** die organischen Abfälle ausgewählt sind aus Hydrolysaten von Bioabfällen, hydrolysierten Schlämmen aus Kläranlagen, verschiedenen organischen flüssigen Fraktionen von Kläranlagen, städtischen Abwässern nach der ersten Klärung, organischen Industrieabwässern, Lebensmittelindustrieabwässern, Rückständen von Kläranlagen oder einer Mischung von mehreren derselben.

## Claims

1. A bio-electrochemical reactor (1) comprising:
- an anode compartment (11) comprising at least two anodes (12, 13) called bioanodes, and an anodic electrolyte (14) comprising anodic electroactive microorganisms;
- a cathode compartment (21) comprising at least one cathode called biocathode (22), and a cathodic electrolyte (24) comprising cathodic electroactive microorganisms;
- the anode compartment (11) being separated from the cathode compartment (21) - from the anode compartment towards the cathode compartment - by a cation exchange membrane (31) and anion exchange membrane (32), said cation and anion exchange membranes being separated from each other by an inter-membrane compartment (30);
- means for applying a potential difference between the bioanodes connected together and the bio-cathodes(s),
the bioanodes and biocathode(s) having active surface areas such that the total active surface area of the biocathode(s) (22) is greater than the total active surface area of the two bioanodes (12, 13).

2. The reactor according to claim 1, **characterized in that** the bioanodes (12, 13) are removable.

3. The reactor according to claim 1 or 2, **characterized in that** said reactor is a microbial electrosynthesis reactor, the anode compartment (11) comprising one or more ports (3) for injecting organic carbon substrate such as hydrolysates of organic bio-wastes, the cathode compartment (21) comprising one or more ports for injecting CO₂ or for feeding an organic or mineral carbon source, and the inter-membrane compartment (30) comprising a device for withdrawing molecules synthesized within said reactor.

4. The reactor according to any of the preceding claims, **characterized in that** the biocathode (22) is an electrode having geometric thickness/height/width dimensions such that the thickness thereof corresponds to its smallest dimension and is greater than or equal to 1/10 of each of its two other dimensions.

5. The reactor according to claim 4, **characterized in that** the biocathode (22) comprises a granular material or is in the general form of a mesh.

6. The reactor according to claim 4, **characterized in that** the biocathode (22) comprises granular carbon arranged in a stainless-steel container.

7. The reactor according to any of the preceding claims, **characterized in that** the bioanodes (12, 13) are in the general shape of a panel, in particular planar or rounded, or by general panel shape it is meant an electrode having a thickness less than 1/10 of each of its two other dimensions, height and width.

8. The reactor according to claim 7, **characterized in that** the bioanodes (12, 13) are formed from carbon cloth or carbon felt held within a metal frame, preferably a frame in stainless steel.

9. The reactor according to any of the preceding claims, **characterized in that** it comprises means for adjusting pH, temperature and/or electrolyte level.

10. A method for regenerating the activity of the bioanodes of the reactor according to any of claims 2 to 9, comprising:
- a step to remove at least one of the bioanodes (12, 13) from the anode compartment (11), on the understanding that at least one bioanode is left in the anode compartment;
- a step, outside the reactor (1) to clean said removed bioanode(s); then
- replacing the latter in the anode compartment (11)
the reactor being maintained in operation by applying a potential difference between the biocathode and the bioanode remaining the anode compartment.

11. A method for regenerating the activity of the bioanodes of the reactor according to any of claims 2 to 9, comprising:
- replacing at least one of the bioanodes (12, 13) of the anode compartment (11) by an anode non-colonized by electroactive microorganisms, on the understanding that at least one bioanode is left in the anode compartment,
the reactor being maintained in operation by applying a potential difference between the biocathode and the bioanode remaining in the anode compartment.

12. Use of the reactor according to any of claims 1 to 9 for electrosynthesis of organic acids and/or alcohols from organic waste.

13. The use according to claim 12, **characterized in that** the organic waste is selected from among: hydrolysates of biowaste, hydrolysed sludge from wastewater treatment plants, different organic liquid fractions from wastewater treatment plants, municipal wastewater after primary decantation, organic industrial effluent, effluent from agri-food industries, digestates from wastewater treatment plants, or a mixture of several of these.
